# EUROPÄISCHE PATENTANMELDUNG

(11) **EP 2 497 521 A1**
(43) Veröffentlichungstag der Anmeldung: **12.09.2012**
(21) Anmeldenummer: 11075043.7
(22) Anmeldetag: 10.03.2011
(51) Int. Cl.: A61M 25/01

(54) **Schubvorrichtung zum axialen Einschieben eines strangförmigen, flexiblen Körpers**

(71) Anmelder: ECP Entwicklungsgesellschaft mbH, 12247 Berlin (DE)
(72) Erfinder: Schulz, Heike, 12437 Berlin (DE); Röhn, Daniel, 12437 Berlin (DE)
(74) Vertreter: Pfenning, Meinig & Partner GbR

(57) **Zusammenfassung**

Bei einer Schubvorrichtung zum axialen Einschieben eines strangförmigen, flexiblen Körpers (8, 10), insbesondere eines Schlauches, eines Seils oder ähnlicher Elemente in eine Schleuse ist eine Axialkrafteinleitungseinheit (19, 20, 47, 49) vorgesehen, die durch Kraft- oder Formschluss eine Schubbewegung auf den strangförmigen Körper aufbringt und die relativ zu der Schleuse (2) ortsfest angeordnet oder auf einer vorgegebenen Bewegungsbahn geführt ist.

## Beschreibung

Die Erfindung liegt auf dem Gebiet der Mechanik, insbesondere des Maschinenbaus bzw. der Feinwerktechnik. Sie befasst sich mit den mechanischen Problemen, die bei einem Einschieben eines flexiblen strangförmigen Körpers in eine Öffnung bzw. eine Schleuse aufgrund seiner Tendenz zum Ausknicken entstehen.

Derartige flexible strangförmige Körper können beispielsweise Seile, Drähte, Fäden, Schläuche oder eine Kombination solcher Elemente sein, die typischerweise in Schleusen oder Öffnungen, Rohre, Schläuche oder allgemein Hohlräume eingeschoben werden sollen. Ein solcher strangförmiger Körper kann auch eine zweite Schleuse sein, die in eine erste Schleuse eingeschoben oder dieser gegenüber ortsfest gehalten werden soll. Wenn sich beim Schieben solcher Körper ein Widerstand gegen eine weitere Schubbewegung ergibt, so muss der Körper, um eine weitere Schubbewegung zu ermöglichen, am Ausknicken gehindert werden, oder entsprechende radiale Ausweichbewegungen müssen begrenzt werden.

Insgesamt geht es unter anderem darum, dass die Handhabung so sicher wie möglich gestaltet wird. Vorteilhaft ist es dabei, wenn beim Einführen ein bestimmter Knickwinkel nicht überschritten und ein bestimmter Biegeradius nicht unterschritten wird.

Typischerweise ergeben sich derartige Probleme unter anderem in der Medizintechnik, wenn beispielsweise eine Kanüle oder ein Katheter in eine Körperöffnung, ein körpereigenes Lumen oder ein artifizielles Lumen eingeschoben werden soll.

Typische derartige Situationen sind beispielsweise aus der internationalen Patentanmeldung WO 02/43791 A1 ersichtlich. Dort ist beschrieben, dass eine intravasale Pumpe in eine Schleuse eingeführt werden kann, um sie letztendlich in ein Blutgefäß eines menschlichen Körpers einzuschieben. Eine weitere Schubbewegung findet statt, auch nachdem die Pumpe den eigentlichen Schleusenbereich passiert hat.

Da derartige Pumpen über Längen von einigen zehn Zentimetern durch ein Blutgefäß innerhalb eines Körpers geschoben werden sollen, ist mit einem gewissen Schubwiderstand zu rechnen, der eine entsprechende Schubkraft beim Einschieben notwendig macht. Insbesondere bei selbstentfaltbaren bzw. selbstexpandierbaren Pumpenköpfen ist aufgrund der auftretenden Rückstellkräfte des gefalteten bzw. komprimierten Pumpenkopfes mit einem erhöhten Schubwiderstand zu rechnen. Besonders deshalb, weil bei derartigen Pumpen innerhalb eines zu schiebenden Katheters eine für hohe Drehzahlen und eine lange Standzeit vorgesehene biegsame Welle verläuft, die knickempfindlich ist, muss sichergestellt werden, dass die notwendige Schubkraft auf den Katheter angewendet werden kann, ohne dass dieser ausknickt oder anderweitig ausweicht.

Der vorliegenden Erfindung liegt die Aufgabe zugrunde, eine Schubvorrichtung zum axialen Einschieben eines strangförmigen, flexiblen Körpers in eine Schleuse oder zur sicheren Halterung eines solchen Körpers gegenüber einer Schleuse zu schaffen, die möglichst einfach aufgebaut ist, die einfache Erzeugung einer Schubbewegung ermöglicht und ein Ausweichen des strangförmigen Körpers zuverlässig verhindert.

Die Aufgabe wird gemäß der Erfindung mit den Merkmalen des Patentanspruchs 1 gelöst.

Dazu ist eine Halteeinheit vorgesehen, die als Axialkrafteinleitungseinheit ausgebildet sein kann, die durch Kraft- oder Formschluss eine axiale Halte- oder Schubkraft auf den strangförmigen Körper aufbringt und die relativ zu der Schleuse ortsfest angeordnet oder auf einer vorgegebenen Bewegungsbahn geführt ist. Unter der axialen Richtung soll in diesem Zusammenhang die Längsachsenrichtung der Schleuse und/oder des strangförmigen Körpers verstanden werden.

Da oft nicht unmittelbar an einem Ende des strangförmigen Körpers angegriffen werden kann, muss eine Axialkrafteinleitungseinheit entweder durch Klemmen, d. h. Kraftschluss und entsprechende Haftreibungskräfte, oder durch einen Formschluss an dem strangförmigen Körper angreifen, sofern dieser entsprechende Formelemente an der Außenseite aufweist. Ein entsprechendes Verklemmen oder ein Formschluss erlaubt dann das Aufbringen einer Axialkraft und damit eine Haltekraft oder eine Schubbewegung. Beispielsweise kann vorgesehen sein, dass sowohl die (erste) Schleuse als auch der strangförmige Körper, der ja auch als (dann zweite) Schleuse ausgebildet sein kann, jeweils einen Flansch aufweisen, wobei jeder der Flansche in je eine Ausnehmung der Schubvorrichtung oder beide Flansche gemeinsam in eine einzige Ausnehmung der Schubvorrichtung derart einlegbar sind, dass die Flansche in Axialrichtung und/oder Radialrichtung festgelegt sind.

Die Schubvorrichtung kann zum Einlegen des Flansches / der Flansche in die Ausnehmung(en) demontierbar sein und beispielsweise als längsgeteilter Hohlkörper, insbesondere Hohlzylinder ausgebildet sein.

Dadurch, dass in der Schubvorrichtung die Halteeinheit/Axialkrafteinleitungseinheit ortsfest zur (ersten) Schleuse angeordnet ist, kann der Abstand und die Ausrichtung der Einheit derart gewählt werden, dass die Axialbelastung des strangförmigen Körpers gut kontrollierbar und begrenzbar ist, dass nur unschädliche Radialkräfte und Radialauslenkungen auf den strangförmigen Körper aufgebracht werden und dass die freie Länge des strangförmigen Körpers zwischen der Axialkrafteinleitungseinheit und der Schleuse definiert bzw. begrenzt werden kann.

Auch wenn die Axialkrafteinleitungseinheit auf einer vorgegebenen Bewegungsbahn geführt ist, kann diese entsprechend so gewählt werden, dass die Tendenz des strangförmigen Körpers, bei einer Schubbewegung seitlich auszubrechen, minimiert ist. Zudem kann eine mechanische Führung für den strangförmigen Körper bei einer vorgegebenen Bewegungsbahn der Axialkrafteinleitungseinheit gut dimensioniert und positioniert werden.

Die Führung der Axialkrafteinleitungseinheit kann beispielsweise mittels einer ein- oder mehrstufigen teleskopartigen Führung, beispielsweise mit konzentrischen Rohren, oder durch Führung in einer Schiene oder einem zylindrischen Hohlraum realisiert sein.

Vorteilhaft kann vorgesehen sein, dass die Schubvorrichtung unmittelbar mechanisch an die Schleuse gekoppelt ist. Dies kann beispielsweise durch ein Zentralgewinde, einen Bajonettverschluss, durch die Anwendung von Adhäsionskräften, mittels einer magnetischen Kopplung, mittels exzentrischer Verschraubung (beispielsweise durch am Umfang verteilte Schrauben), durch Kleben, durch elektrostatische Anziehungskräfte, durch elastische Halteelemente wie beispielsweise Federn, Klammern oder Gummizüge oder durch Einlegen eines Passstückes der Schleuse in eine passend geformte Ausnehmung der Halteeinheit erreicht werden.

Vermieden werden soll insbesondere ein Ausweichen des strangförmigen Körpers beim Eintritt in die Schleuse, da in der Schleuse und in dem zur Schleuse gehörigen Schlauch eine mechanische Führung und radiale Stützung üblicherweise gegeben ist oder einfach hergestellt werden kann und hinter der Schleuse bzw. dem zur Schleuse gehörigen Schlauch aufgrund der verminderten Rückstellkräfte das Knickrisiko eine untergeordnete Rolle spielt.

Durch die Kopplung der Schubvorrichtung an die Schleuse wird der Abstand zwischen der Halteeinheit/Axialkrafteinleitungseinheit und der Schleuse begrenzt, und es wird eine definierte Führung des strangförmigen Körpers und/oder eine radiale Stützung ermöglicht. Es ist auch besonders einfach, die Bewegungsbahn der Axialkrafteinleitungseinheit, falls eine solche vorgesehen ist, in Bezug auf die Schleuse festzulegen.

Zur Kopplung kann die Schleuse beispielsweise einen Flansch oder eine ebene Frontplatte mit einer Öffnung aufweisen, in die der strangförmige Körper eingeführt werden soll, wobei an die Frontplatte bzw. den Flansch ein entsprechender Gegenflansch der Schubvorrichtung angekoppelt werden kann. Dabei können übliche lösbare Verbindungstechniken angewendet werden oder auch die üblichen Fügetechniken wie Kleben oder andere adhäsive Wirkungen, die vorteilhaft jedoch eine lösbare Verbindung herstellen sollen.

Sollen durch die Schubvorrichtung lediglich die erste Schleuse und ein strangförmiger Körper, beispielsweise eine zweite Schleuse, axial gegeneinander gedrückt werden, so können auch ein Flansch oder eine Frontplatte der ersten Schleuse einerseits und ein Flansch der zweiten Schleuse oder eines anderen strangförmigen Körpers gegeneinander fixiert sein.

Es kann aber auch beispielsweise eine Verbindung durch Ineinanderstecken geschaffen werden, die durch Kraft- bzw. Formschluß gehalten wird. Wichtig ist hierbei im Wesentlichen, dass die Kräfte zum Halten der Verbindung größer sind als die Kräfte, die zum Schieben des strangförmigen Körpers erforderlich sind, damit die Verbindung nicht beim Schieben des strangförmigen Körpers ungewollt gelöst wird.

Eine mechanische Kopplung kann vorteilhaft auch unmittelbar zwischen der Axialkrafteinleitungseinheit und der Schleuse vorgesehen sein. Dadurch kann eine Radialführung zwischen der Axialkrafteinleitungseinheit und der Schleuse minimiert oder ganz weggelassen werden. Die Baugröße der Schubvorrichtung kann damit auch gering gehalten werden.

Vorteilhaft kann die Schubvorrichtung eine an der Schleuse mittels der Kopplung befestigbare Führungseinrichtung aufweisen, die den strangförmigen Körper radial stützt, wobei die Axialkrafteinleitungseinheit relativ zu der Führungseinrichtung entweder ortsfest angeordnet oder auf einer vorgegebenen Bewegungsbahn geführt ist. In diesem Fall ist zwischen der Axialkrafteinleitungseinheit und der Schleuse die Führungseinrichtung vorgesehen, die entweder dazu dient, einen Abstand zwischen der Axialkrafteinleitungseinrichtung und der Schleuse zu überbrücken und damit die Aufbringung einer Schubkraft handhabbar zu machen und/oder bei einem an sich geringen Abstand zwischen der Axialkrafteinleitungseinheit und der Schleuse diesen durch die Führungseinrichtung derart zu gestalten, dass der strangförmige Körper auf dieser Distanz keinesfalls radial ausweichen kann.

Die Axialkrafteinleitungseinheit kann vorteilhaft eine Radialklemmeinrichtung aufweisen, die durch Anpressen eines Klemmwerkzeuges an den strangförmigen Körper in Radialrichtung eine Haftung erzeugt, die zum Aufbringen einer Schub- oder Haltekraft in Axialrichtung dient. Das Klemmwerkzeug kann beispielsweise beweglich ausgestaltet sein und an dem strangförmigen Körper abrollen.

Speziell kann/können als Klemmwerkzeug wenigstens ein, insbesondere zwei, drei oder vier Reibräder vorgesehen sein, die einander wenigstens teilweise in Bezug auf den strangförmigen Körper radial gegenüberliegen. Der strangförmige Körper kann dann zwischen den Reibrädern eingeklemmt sein und durch diese radial gestützt werden. Durch einen Antrieb der Reibräder kann eine Schubbewegung auf den strangförmigen Körper aufgebracht werden. Es können auch mehrere Sätze von Reib- oder Rändelrädern in Längsrichtung des strangförmigen Körpers hintereinander angeordnet werden.

Es kann auch vorgesehen sein, dass die Reibräder, oder wenigstens eines von ihnen, eine Freilaufeinrichtung aufweisen, die eine Rotation in jeweils nur einer Richtung erlaubt, so dass beispielsweise bei Vorschieben der Schubvorrichtung die Reibräder sperren und den strangförmigen Körper schieben und bei Zurückziehen der Schubvorrichtung die Reibräder derart rotieren, dass die Schubvorrichtung gegenüber dem strangförmigen Körper kraftfrei verschoben werden kann. Damit kann schrittweise der strangförmige Körper beispielsweise von Hand oder mittels eines anderen Antriebs durch Verschieben der Schubvorrichtung geschoben werden.

Derartige Antriebe können beispielsweise durch Elektromotoren an den Reibrädern vorgesehen sein, so dass die Schubvorrichtung ortsfest stillstehen kann. Damit ergibt sich auch ein verringerter Platzbedarf bei Anwendung der Schubvorrichtung.

Allgemein kann es sinnvoll sein, dass die Reibräder bezüglich ihrer Rotation steuerbar sind, d. h. entweder aktiv antreibbar oder beispielsweise von außen gesteuert sperrbar. Der kontinuierliche Antrieb der Schubvorrichtung mittels eines Rotationsantriebs der Reibräder lässt beispielsweise auch die Steuerung der eingeleiteten Schubkraft in Abhängigkeit vom Vorschub, der Vorschubgeschwindigkeit oder einer erfassten Gegenkraft zu. Damit kann verhindert werden, dass beispielsweise ein Katheter, der in einen Körper eingeschoben wird, in dem Fall, dass ein erhöhter Widerstand durch Anstoßen an Gefäßwände entsteht, einfach weitergeschoben wird. Eine entsprechende Steuerung kann den Schubvorgang stoppen oder verlangsamen. Eine entsprechende Steuerung, die elektronisch ausgestaltet sein kann, kann entweder die Schubkraft oder die Schubgeschwindigkeit entweder nach einer vorgegebenen Kurve/Charakteristik oder in Abhängigkeit vom Schubwiderstand steuern.

Es kann auch vorteilhaft vorgesehen sein, dass das Klemmwerkzeug mit Klemmbacken radial beiderseits des strangförmigen Körpers versehen ist und dass die Klemmbacken mit dem zwischen ihnen festgelegten strangförmigen Körper im Wesentlichen in dessen Axialrichtung gegenüber der Schleuse und/oder einer Führungseinrichtung geführt bewegbar sind.

Die Klemmbacken können dabei als zwei oder mehr in Radialrichtung bezüglich des strangförmigen Körpers relativ zueinander bewegbare Körper ausgebildet sein, die beispielsweise auch nachgiebig, insbesondere weicher als der strangförmige Körper selbst, ausgebildet sein können. Zu diesem Zweck sollten die Klemmbacken aus einem weichen Material wie beispielsweise Kunststoff oder einem Elastomer bestehen oder mit einem solchen Werkstoff beschichtet sein. Es kann auch vorgesehen sein, dass nur einige Klemmbacken beweglich ausgebildet sind, während andere Klemmbacken ortsfest fixiert sind.

Es können beispielsweise zwei, drei, vier, fünf oder mehr Klemmbacken vorgesehen sein, die einander am Umfang des strangförmigen Körpers gegenüberliegen.

Besonders vorteilhaft können die Klemmbacken durch zwei oder mehr Teile beispielsweise eines Hohlzylinders bzw. einer beliebigen anderen zum strangförmigen Körper korrespondierenden Form gebildet sein, der den strangförmigen Körper aufnimmt. Die Klemmbacken können dann Sektorkörper des Hohlzylinders bilden. Unter einem Hohlzylinder soll in diesem Zusammenhang ein Körper verstanden werden, der eine, insbesondere zylindrische, durchgehende Öffnung aufweist. Ein solcher Körper kann beispielsweise auch auf seiner Außenseite eine zylindrische Form aufweisen. Anstelle eines Hohlzylinders kann auch ein andersartiger Körper mit einer durchgehenden Öffnung verwendet werden.

Die entsprechenden Sektorkörper können dann beispielsweise dieselbe Länge aufweisen wie der Hohlzylinder und am Umfang des strangförmigen Körpers verteilt sein. Die Schubvorrichtung kann auch derart ausgebildet sein, dass erste Klemmbacken in axialer Richtung unbeweglich angeordnet sind und aus einem Material bestehen, das eine geringe Reibung gegenüber dem Material des strangförmigen Körpers aufweist. Zweite Klemmbacken sind dagegen axial beweglich und weisen einen höheren Reibungskoeffizienten gegenüber dem Material des strangförmigen Körpers auf, so dass der strangförmige Körper durch die zweiten Klemmbacken gegen die ersten Klemmbacken gedrückt werden und auf diesen wie auf einer Führungsschiene in axialer Richtung gleitend bewegt werden kann. Auf diese Weise kann durch die Klemmbacken sowohl die radiale Führung für den strangförmigen Körper als auch durch einzelne der Klemmbacken der Vorschub in axialer Richtung realisiert werden.

Ein Verfahren zur Realisierung der Erfindung sieht vor, dass durch eine Steuereinrichtung die Geschwindigkeit des Vorschubs des strangförmigen Körpers gesteuert wird. Dabei kann die Geschwindigkeit als konstante Geschwindigkeit oder auch entsprechend einer vorgegebenen Geschwindigkeits-/Zeit-Charakteristik oder einer Geschwindigkeits-/Weg-Charakteristik angesteuert werden.

Ergeben sich bei der Schubbewegung unvorhergesehene Widerstände für den strangförmigen Körper, beispielsweise wenn dieser in ein Blutgefäß eines Patienten eingeschoben wird, so kann durch die Steuerung die entsprechende Antriebskraft verstärkt werden, um die Vorschubgeschwindigkeit gemäß der vorgesehenen Charakteristik sicherzustellen.

Es kann jedoch auch eine Steuerung vorgesehen werden, die den Vorschubwiderstand oder die Vorschubkraft konstant hält, so dass bei Auftreten erhöhter Schubwiderstände auf den strangförmigen Körper die Antriebskraft auf die Schubvorrichtung gesenkt wird, beispielsweise um ein Ausknicken des strangförmigen Körpers durch die Erhöhung von Schubkräften zu verhindern oder um beim medizinischen Einsatz, beispielsweise wenn der strangförmige Körper ein Katheter ist, der in eine Körperöffnung eingeschoben wird, eine Verletzung des Patienten zu vermeiden. Es kann in diesem Fall durch die Steuerung auch der Vorschub vollständig angehalten und der strangförmige Körper ein Stück weit zurückgezogen werden.

Insofern kann der Schubwiderstand gemessen und die Schubbewegung in Abhängigkeit vom Schubwiderstand gesteuert bzw. geregelt werden. Hierzu sind ein Antrieb, beispielsweise ein elektrischer oder pneumatischer Antrieb, und ein entsprechender Sensor, beispielsweise ein Stromsensor zur Messung der abgenommenen Stromstärke, ein Spannungssensor oder ein entsprechender Drucksensor bei Verwendung einer Pneumatik, sowie eine Steuereinrichtung vorzusehen.

Im Folgenden wird die Erfindung anhand eines Ausführungsbeispiels in einer Zeichnung gezeigt und nachfolgend beschrieben. Dabei zeigt
- Fig. 1: die Anwendung einer Herzkatheterpumpe bei einem menschlichen Herzen,
- Fig. 2: eine Pumpeneinrichtung mit einer Herz-katheterpumpe und einer Antriebswelle, die in einem schlauchförmigen Mantel verläuft,
- Fig. 3: eine Schubvorrichtung in einer dreidimen-sionalen Ansicht,
- Fig. 4: die Schubvorrichtung aus Fig. 3 in einer Liniendarstellung,
- Fig. 5: einen Längsschnitt durch die Vorrichtung aus Fig. 4,
- Fig. 6: eine als Handgriff ausgestaltete Führungs-einrichtung in einem Längsschnitt mit dem vorderen Ende einer Schleuse,
- Fig. 7: eine Führungseinrichtung ohne eingelegte Schleuse,
- Fig. 8: die Führungseinrichtung aus Fig. 7 in einer Frontansicht.
- Fig. 9: eine Schubvorrichtung mit wenigstens zwei Reibrädern,
- Fig. 10: eine Draufsicht auf die Vorrichtung nach Fig. 9 entlang der in Fig. 3 mit IV-IV be-zeichneten Linie,
- Fig. 11: eine ähnliche Ansicht wie Fig. 10 mit vier um den strangförmigen Körper verteilten Reibrädern,
- Fig. 12: eine ähnliche Ansicht wie Fig. 11, zusätz-lich mit elektromechanischen Antrieben der Reibräder,
- Fig. 13: eine Anordnung mit zwei Reibrädern, die im Querschnitt konkave Reibflächen aufweisen,
- Fig. 14: eine Schubvorrichtung ähnlich der aus Fig. 9, bei der die Reibräder nur der radi-alen Stützung dienen und eine andere Art der Axialkrafteinleitung vorgesehen ist,
- Fig. 15: eine Ansicht der Einrichtung aus Fig. 14 entlang der dort mit IX-IX gekennzeichneten Linie,
- Fig. 16: eine weitere Variante der Axialkraft-einleitungseinheit,
- Fig. 17: einen Querschnitt der Axialkraftein-leitungseinheit aus Fig. 16,
- Fig. 18: eine weitere Ausgestaltung der Axialkraft-einleitungseinheit,
- Fig. 19: eine Querschnittsansicht entlang der Linie XIII-XIII aus Fig. 18,
- Fig. 20: eine andere Variante der Axialkraftein-leitungseinheit aus Fig. 18,
- Fig. 21: in einem Längsschnitt eine Schubvorrichtung mit axial feststehenden und axial beweg-lichen Klemmbacken,
- Fig. 22: einen Querschnitt der Einrichtung aus Fig. 21 entlang der dort mit XVI-XVI ge-kennzeichneten Linie und
- Fig. 23: ein Detail einer Antriebseinrichtung für eine Schubvorrichtung.

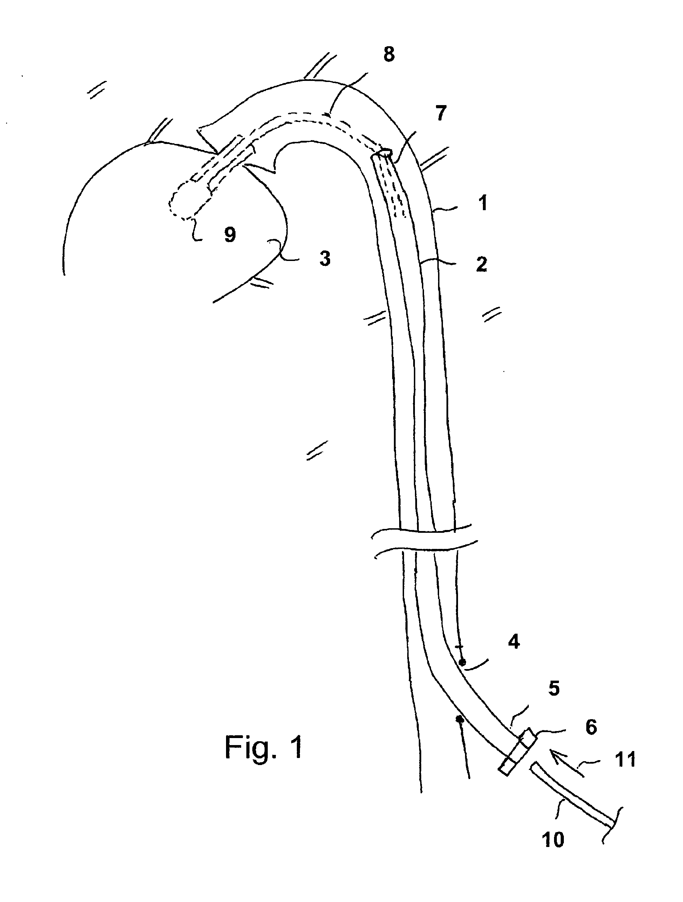

Fig. 1 zeigt in einem Längsschnitt ein Blutgefäß 1 mit einer ersten Schleuse 2 in Schlauchform, die mit ihrem distalen Ende in das Blutgefäß 1 in Richtung einer Herzkammer 3 bis zum Aortenbogen hineinragt. Die Schleuse 2 ist durch eine Öffnung 4 in das Blutgefäß 1 eingeführt und idealerweise dort gedichtet. An ihrem proximalen (körperäußeren) Ende 5 weist die erste Schleuse 2 einen Flansch 6 auf.

Gestrichelt ist am distalen Ende 7 der Schleuse 2 ein Herzkatheter 8 gezeigt, der hohl ausgebildet ist und in dem eine Antriebswelle für eine ebenfalls gestrichelt dargestellte Herzpumpe 9 verläuft. Die Pumpe 9 ist in die Herzkammer 3 eingeschoben und weist einen Rotor auf, der mit hoher Drehzahl, durch die Welle angetrieben, rotiert, um Blut aus der Herzkammer in das Blutgefäß 1 zu fördern.

Am proximalen Ende 5 der Schleuse 2 ist vor dem Flansch 6 ein strangförmiger Körper 10 dargestellt, der in Richtung des Pfeiles 11 in die Schleuse 2 eingeführt werden soll. Der strangförmige Körper 10 ist dort nur abschnittsweise dargestellt. In dem dargestellten Fall ist der strangförmige Körper beispielsweise durch den Herzkatheter 8 gebildet, der mitsamt der darin befindlichen flexiblen Antriebswelle einen flexiblen strangförmigen Körper darstellt und der durch die Schleuse 2 in das Blutgefäß 1 und weiter in die Herzkammer 3 eingeschoben werden soll. Je nachdem, an welchem Punkt der Herzkatheter in ein Blutgefäß eingeschoben wird (typischerweise an einer Femoralarterie), beträgt der Vorschubweg für den Katheter mehr als 80 cm im Patientenkörper.

Der strangförmige Körper 10 kann auch aus einem Herzkatheter 8 bestehen, der von einer zweiten, schlauchförmigen Schleuse umgeben ist. Diese zweite Schleuse ist dann so ausgebildet, dass sie die radial komprimierbare Pumpe komprimiert hält. Die zweite Schleuse kann vor der Verwendung an einem Patienten zweckmäßigerweise schon mit dem Herzkatheter 8 und der Pumpe vorkonfektioniert werden.

Die zweite Schleuse kann dann axial an die erste Schleuse gehalten und dort fixiert werden, beispielsweise mittels der erfindungsgemäßen Schubvorrichtung. Während die zweite Schleuse axial gegen die erste Schleuse gepresst wird, kann dann der Herzkatheter durch die zweite Schleuse hindurch in die erste Schleuse hinein und durch diese in das Blutgefäß geschoben werden.

Beim axialen Einschieben des Herzkatheters in die zweite Schleuse kann ebenfalls eine erfindungsgemäße Schubvorrichtung Anwendung finden. Diese wird dann an die zweite Schleuse gegenüber der ersten Schleuse angesetzt und umschließt den Abschnitt des Herzkatheters vor der zweiten Schleuse.

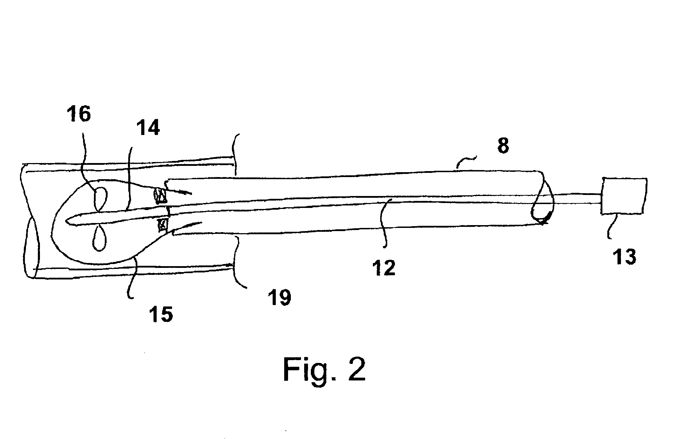

Fig. 2 zeigt eine detailliertere, aber schematische Darstellung des Pumpenkatheters 8 mit der flexiblen Antriebswelle 12. Diese ist proximal mit einem Antrieb in Form eines Motors 13 und distal mit einer Antriebsnabe 14 innerhalb eines Pumpengehäuses 15 verbunden. Die Nabe 14 trägt Rotorblätter 16 zur Förderung eines Fluids bzw. von Blut.

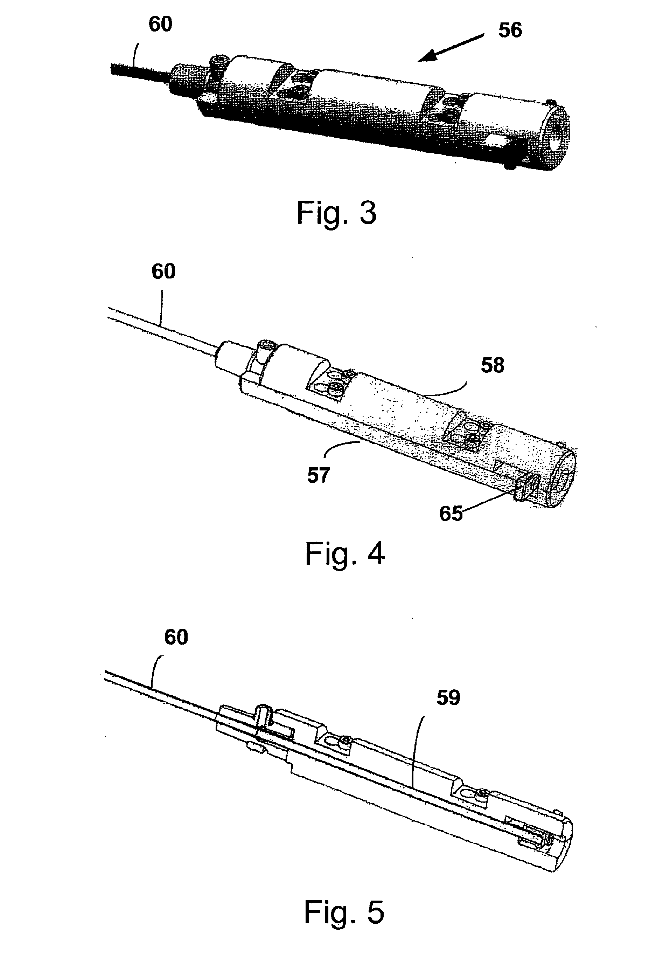

Die Fig. 3 zeigt in dreidimensionaler Ansicht eine Konstruktion einer Schubvorrichtung.

Fig. 4 zeigt dieselbe Ansicht als Strichzeichnung und Fig. 5 einen Längsschnitt.

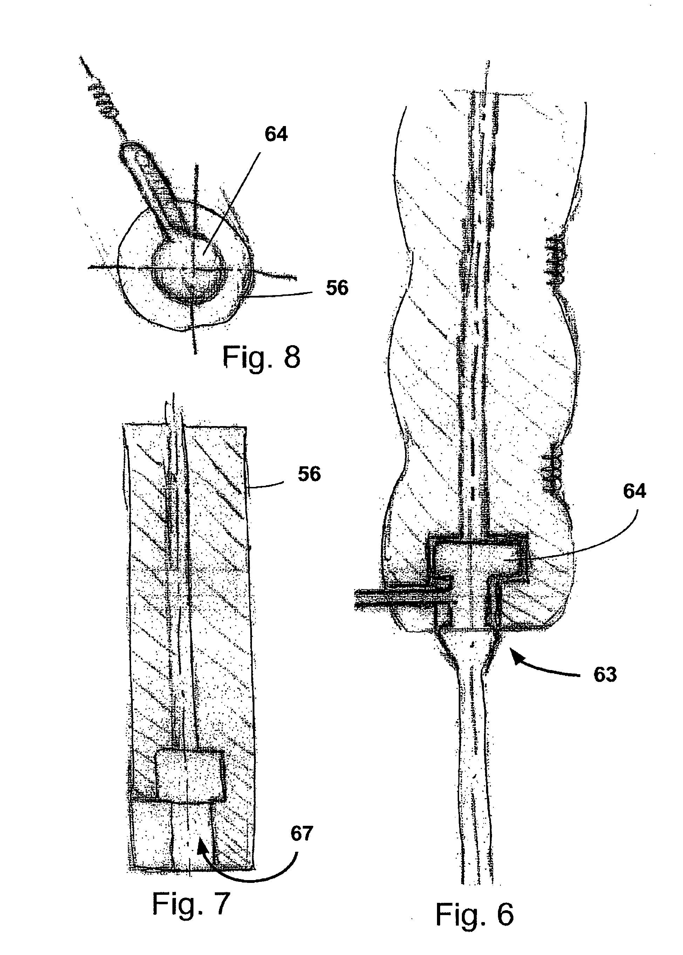

Es ist eine Führungseinrichtung 56 in Form eines zweigeteilten Hohlzylinders mit einer unteren Hälfte 57 und einer oberen Hälfte 58 dargestellt. Der zylindrische Hohlraum 59 nimmt den zu schiebenden strangförmigen Körper 60, sowie eine zweite Schleuse, auf und stützt ihn radial. Die obere Hälfte 58 ist über Schrauben 61, 62 mit der unteren Hälfte 57 verbunden, die in Langlöchern gleiten. Die Langlöcher sind an einem Ende erweitert, so dass nach Verschieben der oberen Hälfte diese abgenommen werden kann. Dadurch kann der strangförmige Körper mit der zweiten Schleuse ebenso wie die erste Schleuse mit ihrem Flansch 64 problemlos eingelegt werden, und das frontseitige Ende 63 der Schleuse (vgl. Fig. 6), insbesondere der Flansch 64, kann in den hinterschnittenen Hohlraum 67 des Hohlzylinders 56 eingefügt werden (Figuren 6, 7). Hierdurch wird in einfacher Weise eine zuverlässige lösbare Kopplung der Führungseinrichtung an die Schleuse sichergestellt.

Eine Halteeinheit/Axialkrafteinleitungseinrichtung kann Ausnehmungen für Reißlaschen 65 einer zweiten Schleuse aufweisen, wenn die zweite Schleuse als sogenannte "Peel-off"-Schleuse ausgebildet ist. Das bedeutet, dass die zweite Schleuse durch ihre Materialstruktur (Molekularausrichtung, anisotrope Struktur) und/oder das Einbringen von Sollbruchstellen (Perforierung) dafür eingerichtet ist, nach dem Durchschieben eines Herzkatheters längs aufgerissen und entfernt zu werden.

Fig. 8 zeigt zur weiteren Illustration einen Querschnitt aus Fig. 6.

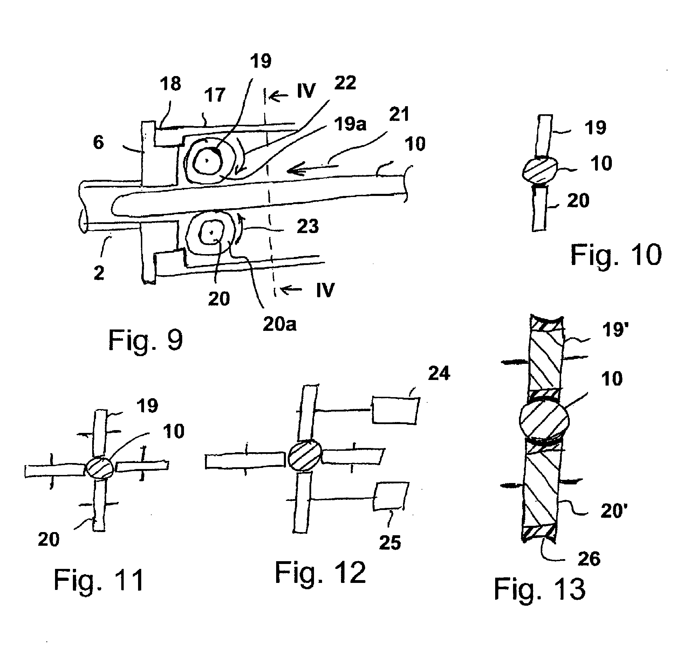

Fig. 9 zeigt konkret eine andere Ausführungsform einer Vorschubeinrichtung, wobei der Flansch 6 der ersten Schleuse 2 auf der linken Seite und die Schubvorrichtung im rechten Bereich der Figur dargestellt ist. Die Schubvorrichtung weist ein rohrförmiges Gehäuse 17 auf, dessen distales Ende 18 auf den Flansch 6 aufschiebbar und dort gegenüber dem Flansch 6 fixiert ist. Die Fixierung kann beispielsweise mittels eines Zentralgewindes an dem Flansch 6 sowie an dem Gehäuseende 18 oder durch einen Bajonettverschluss zwischen diesen Teilen realisiert sein. Es sind auch verschiedene Arten von Schraub- und anderen Haftverbindungen wie Kleben oder magnetische Haftung denkbar. Sinnvoll ist, wenn die Verbindung zwischen der Schubvorrichtung und dem Flansch 6 der Schleuse leicht wieder lösbar ist. Die Schubvorrichtung kann auch einen Hohlraum zur Aufnahme und Fixierung des Flansches 6 aufweisen sowie eine Öffnung zum Einbringen des Flansches in den Hohlraum.

Innerhalb des Gehäuses 17 sind wenigstens zwei Reibräder 19, 20 als Teil einer Axialkrafteinleitungseinrichtung oder Halteeinheit drehbar gelagert, die jeweils an ihrem Umfang einen Reibbelag 19a, 20a aufweisen, der einen hohen Reibungskoeffizienten gegenüber dem Material des strangförmigen Körpers 10 aufweist. Der strangförmige Körper 10 ist typischerweise zwischen den Reibrädern 19, 20 eingeklemmt. Der strangförmige Körper kann dabei sowohl ein Katheter als auch eine zweite Schleuse sein.

Die Reibräder 19, 20 können beispielsweise in ihrer Drehbarkeit steuerbar sein. Werden sie blockiert, so kann durch axiale Bewegung der Schubvorrichtung mitsamt den Reibrädern, wenn diese in dem Gehäuse 17 geführt bewegbar sind, insgesamt der strangförmige Körper in Richtung des Pfeiles 21 vorgeschoben werden. Wird die Schubvorrichtung entgegen der Richtung des Pfeils 21 zurückgezogen, so können die Reibräder 19, 20 beispielsweise durch eine Steuerung oder einen Freilauf freigegeben werden, so dass bei dieser Bewegung der Schubvorrichtung der strangförmige Körper 10 nicht mitgenommen wird, sondern in der Position relativ zur Schleuse 2 verbleibt. Vor einem erneuten Vorschieben der Schubvorrichtung in Richtung des Pfeils 21 können die Reibräder 19, 20 wieder blockiert werden. Beispielsweise kann zur Realisierung eines solchen Antriebsmechanismus das Gehäuse 17 auf einem Stutzen des Flansches 6 axial vor- und zurückverschiebbar sein.

Eine andere Variante eines Antriebs kann vorsehen, dass die Reibräder 19, 20 bei feststehender Schubvorrichtung in Richtung der Pfeile 22, 23 antreibbar sind, um den strangförmigen Körper 10 in Richtung des Pfeils 21 zu schieben. Diese Variante hat den Vorteil, dass die Geschwindigkeit der Reibräder 19, 20 oder das Drehmoment, beispielsweise anhand des Stroms eines die Reibräder antreibenden Elektromotors, erfasst werden kann. Die entsprechenden erfassten Größen können zur Steuerung der Vorschubgeschwindigkeit des strangförmigen Körpers oder zur Steuerung der Schubkraft verwendet werden, um diese zu begrenzen und damit Verletzungen beim Einschieben eines Katheters in ein Lumen innerhalb eines Patientenkörpers zu vermeiden. Alternativ kann die Schubkraft beispielsweise auch durch eine Rutschkupplung begrenzt werden.

Fig. 10 zeigt in einer Draufsicht entlang der Linie IV-IV gemäß Fig. 3 die beiden Reibräder 19, 20 sowie den zwischen ihnen eingeklemmten strangförmigen Körper 10.

In der Fig. 11 ist dargestellt, dass durchaus auch mehr als zwei, beispielsweise vier Reibräder 19, 20 am Umfang des strangförmigen Körpers 10 verteilt vorgesehen werden können. Es können dies beispielsweise auch drei, fünf oder sechs Reibräder sein.

Für jedes einzelne Reibrad ist eine drehbare Lagerung anhand einer stilisierten Welle schematisch dargestellt. Die Reibräder können beispielsweise mittels einzelner motorischer Antriebe oder gemeinsam über ein Koppelgetriebe antreibbar sein. Es kann auch vorgesehen sein, dass nur einzelne der Reibräder oder ein einziges von ihnen antreibbar ist. Die übrigen Reibräder würden sich in diesem Fall beim Antrieb eines einzigen Reibrades selbsttätig mitdrehen.

Fig. 12 zeigt ein Beispiel einer Implementierung eines Antriebs über zwei Elektromotoren 24, 25.

In den Figuren 4 bis 6 sind die Reibräder schematisch als zylindrische Räder, gegebenenfalls mit einem Reibbelag, dargestellt.

Entgegen dieser Konstruktion kann auch gemäß Fig. 13 eine konkave äußere Umfangsfläche der Reibräder 19', 20' vorgesehen sein. Die konkaven Flächen 26 sind derart gestaltet, dass sie sich an den strangförmigen Körper 10 anschmiegen und diesen auch seitlich halten, so dass bereits mit zwei Reibrädern eine zuverlässige radiale Führung des strangförmigen Körpers gewährleistet werden kann.

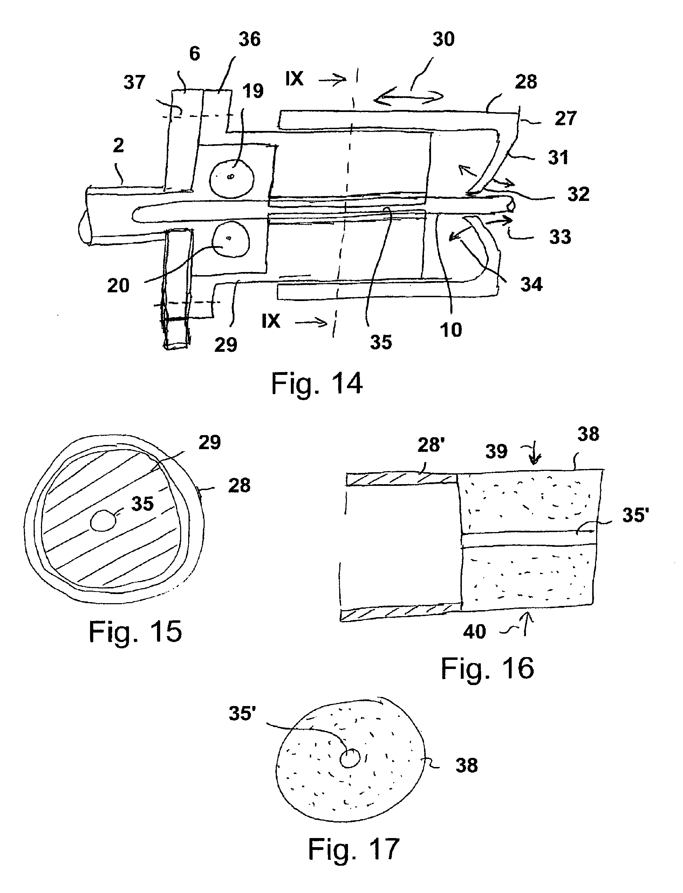

Fig. 14 zeigt eine Vorschubeinrichtung mit einer Axialkrafteinleitungseinheit 27 in Form eines Schubzylinders 28, der auf einem Führungskörper 29 in den Richtungen des Pfeils 30 hin- und herverschiebbar ist. Der Schubzylinder 28 weist an einem Ende eine sickenförmige Scheibe 31 mit einer zentrischen Öffnung auf, die von einer umlaufenden beweglichen Lippe 32 umgeben ist. Die Öffnung ist derart bemessen, dass der Rand der Lippe 32 auf dem strangförmigen Körper 10 aufliegt. Wird der Schubzylinder 28 auf die Schleuse 2 zu geschoben, so wird die Lippe 32 in Richtung des Pfeils 33 ausgelenkt, und die zentrische Öffnung der Sickenscheibe 31 verkleinert sich und klemmt den strangförmigen Körper 10 ein. Dieser wird dann in Richtung zur Schleuse 2 hin mitgenommen und dorthin verschoben.

Bei einer Rückzugbewegung des Schubzylinders 28 bewegt sich die Lippe 32 in Richtung des Pfeils 34, so dass sich die zentrische Öffnung der Sickenscheibe 31 vergrößert und der Schubzylinder 28 gegenüber dem ortsfest verbleibenden strangförmigen Körper 10 zurückgezogen werden kann.

Auf diese Weise kann ruckweise der strangförmige Körper 10 in die Schleuse 2 hineinbewegt werden. Die Bewegung des Schubzylinders 28 ist auf dem Führungskörper 29 geführt, so dass die Bewegungsbahn der Axialkrafteinleitungseinheit festgelegt ist. Der strangförmige Körper ist zudem in einer zylindrischen Öffnung 35 des Führungskörpers 29 geführt und weiter in Richtung zur Schleuse 2 durch zwei oder mehr einander gegenüberliegende frei drehbar bewegliche Reibräder 19, 20. Es kann auch eine Führung durch mehrere axial hintereinander liegende Gruppen von Reibrädern einerseits oder ausschließlich durch eine lange hohlzylindrische Öffnung bis zum Flansch 6 der Schleuse 2 hin vorgesehen sein.

Dem Flansch 6 der Schleuse 2 liegt ein ähnlich gebauter Flansch 36 des Führungskörpers 29 gegenüber, so dass die beiden Flansche 6, 36 durch eine Schraubverbindung, dargestellt durch eine beispielhafte gestrichelte Linie 37, miteinander verbunden werden können. Hierzu können mehrere Schrauben am Umfang der Flansche, insbesondere symmetrisch, verteilt sein.

Ein Querschnitt entlang der Schnittlinie IX-IX in der Fig. 14 ist in der Fig. 15 dargestellt, mit der zentrischen Öffnung 35 in dem Führungskörper 29, der von dem Zylinder 28 der Axialkrafteinleitungseinheit 27 umgeben ist.

Fig. 16 zeigt eine andere Variante einer Axialkrafteinleitungseinrichtung, die beispielsweise in der Schubvorrichtung gemäß Fig. 14 anstelle der Axialkrafteinleitungseinrichtung 27 verwendet werden kann. In Fig. 16 ist ein Schubzylinder 28' dargestellt, der auf dem Führungskörper 29 axial geführt sein kann. Zum Einklemmen des strangförmigen Körpers 10 dient ein Schaumstoffkörper 38, der durch Druck in Richtung der Pfeile 39, 40 radial zusammengedrückt werden kann, um die Öffnung 35' zu komprimieren und den strangförmigen Körper in der Öffnung 35' festzuklemmen. Beispielsweise kann der Schaumstoffzylinder 38 von Hand zusammengedrückt werden, während gleichzeitig eine Schubbewegung in Richtung zur Schleuse 2 hin ausgeführt wird. Damit kann der strangförmige Körper in die Schleuse 2 ein Stück weit eingeschoben werden.

Beim Zurückziehen wird der radiale Druck auf den Schaumstoffzylinder 38 verringert, so dass die Öffnung 35' sich in radialer Richtung elastisch wieder vergrößert und der strangförmige Körper freigegeben wird. Somit kann die Axialkrafteinleitungseinheit dann proximal gegenüber dem strangförmigen Körper zurückgezogen werden.

Fig. 17 zeigt einen Querschnitt durch den Schaumstoffzylinder 38 mit der Öffnung 35'.

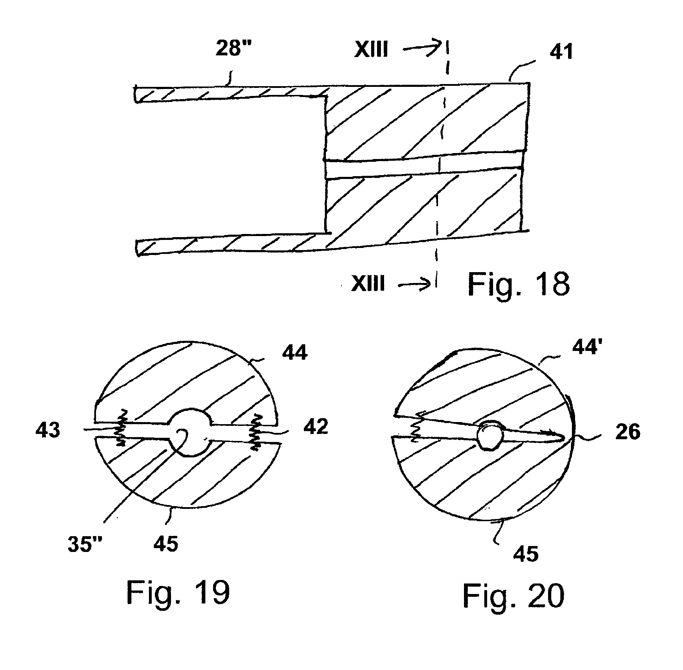

In Fig. 18 ist eine weitere Variante einer Axialkrafteinleitungseinheit dargestellt, mit einem massiven Schubzylinder 41, der ein Führungsrohr 28'' aufweist, das auf dem Führungszylinder 29 wie in Fig. 14 dargestellt geführt werden kann.

In dem Fall, der in der Fig. 18 dargestellt ist, ist der Zylinder 41 selbst aus inkompressiblem Material hergestellt, jedoch in zwei Hälften in Längsrichtung geteilt, so dass sich zwei Segmentkörper des Zylinders 41 ergeben, wie in den Figuren 19 und 20 in verschiedenen Varianten dargestellt. Einmal kann gemäß Fig. 19 der Zylinder 41 gänzlich durchtrennt sein und aus zwei nur durch Federn 42, 43 verbundenen Hälften 44, 45 bestehen. Diese weisen im entspannten Zustand der Federn 42, 43 einen Abstand auf, der so bemessen ist, dass in der zentralen Ausnehmung 35'' der strangförmige Körper 10 mit Spiel Platz findet. Werden die Hälften 44, 45 entgegen der Kraft der Federn 42, 43 zusammengedrückt, so kann der strangförmige Körper in der Öffnung 35'' eingeklemmt werden. Im eingeklemmten Zustand kann dann der strangförmige Körper vorgeschoben werden. Die beiden Hälften 44, 45 des Zylinders 41 können beispielsweise beim Manipulieren der Schubvorrichtung von Hand zusammengedrückt und beim Zurückziehen des Zylinders 41 losgelassen werden.

Fig. 20 zeigt eine ähnliche Ausgestaltungsform, wobei der Zylinder 41 nicht gänzlich in zwei Hälften getrennt ist, sondern ein Filmgelenk 46 an einer Seite des Zylinders 41 verblieben ist, das es erlaubt, die beiden Hälften 44', 45' aufzuklappen. Anstatt des Filmgelenks 26 kann auch ein gesondertes Scharnier vorgesehen sein. Die Funktionsweise beim Klemmen des strangförmigen Körpers entspricht der anhand der Fig. 19 dargestellten.

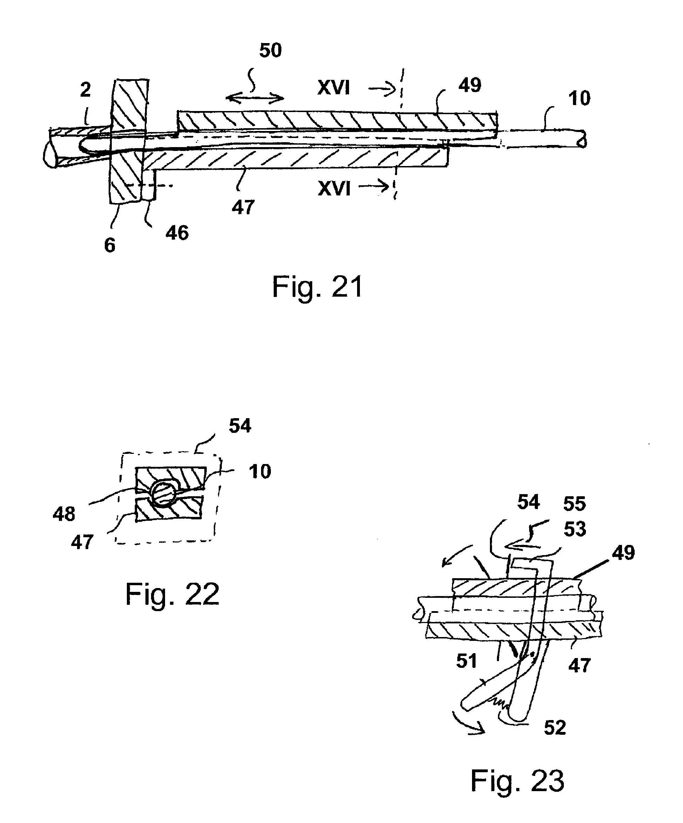

Fig. 21 zeigt eine Schubvorrichtung, die von den in den vorangegangenen Figuren dargestellten Vorrichtungen verschieden ist. Es ist dort an einer Schleuse 2, und zwar genauer an deren Flansch 6, ein Flansch 46 eines Führungskörpers 47 befestigt. Der Führungskörper 47 weist eine Führungsschiene für den strangförmigen Körper 10 auf, die beispielsweise durch eine halbzylindrische oder teilzylindrische Ausnehmung gegeben sein kann, in der der strangförmige Körper 10 liegen kann.

Fig. 22 zeigt im Querschnitt den Führungskörper 47 mit der teilzylindrischen Ausnehmung 48, in der der strangförmige Körper 10 liegt.

Oberhalb des Führungskörpers 47 in der Fig. 21 ist ein Antriebskörper 49 dargestellt, der die Axialkrafteinleitungseinheit bildet. Dieser Antriebskörper 49 weist beispielsweise ebenso eine teilzylindrische Ausnehmung auf, die den strangförmigen Körper 10 wenigstens teilweise aufnimmt. Der Antriebskörper 49 kann in axialer Richtung, dargestellt durch den Doppelpfeil 50, proximal oder distal verschoben werden. Ein besonderes Merkmal des Führungskörpers 47 und des Antriebskörpers 49 kann beispielsweise dadurch realisiert sein, dass die Reibung zwischen dem Antriebskörper 49 und dem strangförmigen Körper 10 durch die Materialwahl mit einem höheren Haftreibungskoeffizienten wesentlich größer ist als die Reibung zwischen dem strangförmigen Körper 10 und dem Führungskörper 47. Beispielsweise kann der Führungskörper 47 aus einem besonders glatten Material mit geringem Reibungskoeffizienten, beispielsweise PTFE, bestehen oder mit einem solchen Material beschichtet sein.

Der Antriebskörper 49 seinerseits kann aus einem Material bestehen, das gegenüber dem strangförmigen Körper 10 einen höheren Reibungskoeffizienten aufweist, beispielsweise einem Elastomer, speziell Gummi oder Silikongummi. Dadurch kann, wenn der Antriebskörper 49 radial gegen den strangförmigen Körper 10 gepresst wird, durch Verschiebung des Antriebskörpers in axialer Richtung der strangförmige Körper 10 axial in der festen Führungsschiene bewegt werden.

Zweckmäßigerweise wird beim Einführen des strangförmigen Körpers 10 in die Schleuse 2 gleichzeitig radial auf den Antriebskörper gedrückt und dieser distal in Richtung auf die Schleuse 2 zu bewegt. Beim Zurückziehen des Antriebskörpers von der Schleuse 2 weg wird die Andruckkraft auf den strangförmigen Körper verringert, so dass dieser nicht mitgenommen wird und in der Schleuse 2 verbleibt. Auf diese Weise kann schrittweise der strangförmige Körper in die Schleuse 2 hineinbefördert werden.

Gemäß Fig. 23 kann an dem Führungskörper 47 auch ein pistolenartiger Handgriff vorgesehen sein, der einen Hebel 51 aufweist, der gegen eine Feder 52 bewegbar ist. Der Hebel 51 ist schwenkbar gelagert und weist einen Stößel 53 auf, der gegen eine Klemmscheibe 54 drückt, diese gegenüber dem Antriebskörper 49 verklemmt und darauf eine Schubbewegung des Antriebskörpers 49 bewirkt. Die Klemmscheibe ist entgegen der Richtung des Pfeils 55 federbelastet.

Die in den Figuren 9-23 gezeigte Vorrichtung kann einerseits für eine zweite Schleuse verwendet werden, um diese als strangförmigen Körper gegen eine erste Schleuse zu drücken und das Durchschieben eines Pumpenkopfes mit minimiertem Knickrisiko des Pumpenkatheters zu ermöglichen. Andererseits kann diese Vorrichtung auch proximal an eine in den Figuren 3-8 gezeigte Haltevorrichtung einer zweiten Schleuse angeschlossen werden, um das knickfreie Einschieben eines Katheters (gegebenenfalls mit Pumpenkopf) in eine zweite Schleuse zu ermöglichen. Auf diese Weise können also zwei Schubvorrichtungen miteinander kombiniert werden.

## Patentansprüche

1. Schubvorrichtung zum axialen Einschieben eines strangförmigen flexiblen Körpers (8, 10), insbesondere eines Schlauches, eines Seils, eines Drahtes oder eines Fadens oder einer Kombination von zwei oder mehr dieser Elemente, in eine Schleuse, **gekennzeichnet durch** eine Halteeinheit (19, 20, 28, 31, 32, 49), die **durch** Kraft- oder Formschluss eine axiale Halte- oder Schubkraft auf den strangförmigen Körper (8, 10) aufbringt und die relativ zu der Schleuse ortsfest angeordnet oder auf einer vorgegebenen Bewegungsbahn relativ zu dieser geführt ist.

2. Schubvorrichtung nach Anspruch 1, **gekennzeichnet durch** eine unmittelbare mechanische Kopplung an die Schleuse.

3. Schubvorrichtung nach Anspruch 2, **dadurch gekennzeichnet, dass** die Schubvorrichtung mittels eines Zentralgewindes, eines Bajonettverschlusses, mittels einer Passfläche durch Adhäsionskräfte, mittels einer magnetischen Einrichtung, mittels einer exzentrischen Verschraubung, mittels einer Klebeverbindung, einer Konusverbindung, einer Schnappverbindung, einer elektrostatischen Verbindung, durch elastische Halteelemente oder durch Einlegen eines Passstückes der Schleuse in eine passend geformte Ausnehmung der Halteeinheit mit der Schleuse verbunden ist.

4. Schubvorrichtung nach Anspruch 2 oder 3, **dadurch gekennzeichnet, dass** die mechanische Kopplung unmittelbar zwischen der Halteeinheit (19, 20, 28, 31, 32, 49) und der Schleuse (2) vorgesehen ist.

5. Schubvorrichtung nach einem der vorangehenden Ansprüche, **gekennzeichnet durch** eine an der Schleuse mittels der Kopplung befestigbare Führungseinrichtung (19, 20, 47), die den strangförmigen Körper radial stützt, wobei die Halteeinheit relativ zu der Führungseinrichtung (19, 20, 47) entweder ortsfest angeordnet oder auf einer vorgegebenen Bewegungsbahn geführt ist.

6. Schubvorrichtung nach Anspruch 1 oder einem der folgenden, **dadurch gekennzeichnet, dass** die Halteeinheit (49) eine radiale Klemmeinrichtung aufweist, die durch Anpressen eines Klemmwerkzeuges an den strangförmigen Körper (8, 10) in dessen Radialrichtung zu diesem eine Haftung erzeugt, die zum Aufbringen einer Schub- oder Haltekraft in Axialrichtung des strangförmigen Körpers dient.

7. Schubvorrichtung nach Anspruch 6, **dadurch gekennzeichnet, dass** das Klemmwerkzeug (19, 20) an dem strangförmigen Körper abrollt.

8. Schubvorrichtung nach Anspruch 7, **dadurch gekennzeichnet, dass** als Klemmwerkzeug wenigstens ein, insbesondere zwei, drei oder vier Reibräder (19, 20) vorgesehen sind, die einander wenigstens teilweise in Bezug auf den strangförmigen Körper (8, 10) gegenüberliegen.

9. Schubvorrichtung nach Anspruch 8, **dadurch gekennzeichnet, dass** die Reibräder (19, 20) bezüglich ihrer Rotation steuerbar sind.

10. Schubvorrichtung nach Anspruch 6, **dadurch gekennzeichnet, dass** das Klemmwerkzeug mit Klemmbacken (47, 49) beiderseits des strangförmigen Körpers versehen ist und dass wenigstens eine der Klemmbacken (49) mit dem zwischen den Klemmbacken festgelegten strangförmigen Körper (8, 10) im Wesentlichen in dessen Axialrichtung gegenüber der Schleuse (2) und/oder einer Führungseinrichtung (47) geführt bewegbar ist.

11. Schubvorrichtung nach Anspruch 10, **dadurch gekennzeichnet, dass** die Klemmbacken (47, 49) durch zwei oder mehr Teile eines in Segmentkörper geteilten Hohlzylinders gebildet sind, der den strangförmigen Körper (8, 10) aufnimmt.

12. Verfahren zum Betrieb einer Schubvorrichtung nach einem der Ansprüche 1 bis 11, **dadurch gekennzeichnet, dass** die Geschwindigkeit des Vorschubs des strangförmigen Körpers (8, 10) gesteuert wird.

13. Verfahren zum Betrieb einer Schubvorrichtung nach Anspruch 12, **dadurch gekennzeichnet, dass** die Schubkraft und die Schubbewegung in Abhängigkeit vom Schubwiderstand des strangförmigen Körpers (8, 10) gesteuert wird.
